(19) Europäisches Patentamt
European Patent Office
Office européen des brevets

(11) **EP 1 448 143 B1**

(12) **EUROPEAN PATENT SPECIFICATION**

(45) Date of publication and mention
of the grant of the patent:
**03.09.2008 Bulletin 2008/36**

(51) Int Cl.:
***A61F 13/514*** (2006.01)

(21) Application number: **02804447.7**

(22) Date of filing: **26.11.2002**

(86) International application number:
**PCT/US2002/037882**

(87) International publication number:
**WO 2003/047489 (12.06.2003 Gazette 2003/24)**

(54) **HIGHLY BREATHABLE WATER RESISTANT COMPOSITE**

STARK ATMUNGSAKTIVER WASSERFESTER VERBUNDSTOFF

COMPOSITE PRESENTANT UNE RESISTANCE A L'EAU ET UN POUVOIR RESPIRANT ELEVES

(84) Designated Contracting States:
**AT BE BG CH CY CZ DE DK EE ES FI FR GB GR IE IT LI LU MC NL PT SE SK TR**

(30) Priority: **29.11.2001 US 334451 P**

(43) Date of publication of application:
**25.08.2004 Bulletin 2004/35**

(73) Proprietor: **Tredegar Film Products Corporation Richmond, VA 23225 (US)**

(72) Inventors:
• **CREE, James, W.**
  **Chesterfield, VA 23838 (US)**
• **IULIANETTI, Lino**
  **I-65029 Torre dei Passeri (IT)**
• **SPLENDIANI, Antonietta**
  **I-65125 Pescara (IT)**

(74) Representative: **Hayes, Adrian Chetwynd et al Boult Wade Tennant, Verulam Gardens 70 Gray's Inn Road London WC1X 8BT (GB)**

(56) References cited:
EP-A- 0 895 766          EP-A- 1 040 806
EP-A- 1 118 339          WO-A- 99/12734
US-A- 5 571 096          US-A- 5 762 643

**Description**

CROSS REFERENCE TO EARLIER APPLICATIONS

This patent application claims priority from United States Provisional Patent Application No. 60/334,451, filed on November 29, 2001 entitled "Wear Resistant High Breathable Laminate" and a United States Patent Application filed on November 26, 2002 entitled "Highly Breathable Water Resistant Composite".

**BACKGROUND OF THE INVENTION**

Technical Field of the Invention

This invention relates to web materials and more particularly to highly breathable and water resistant materials for use in absorbent articles and clothing articles.

Description of Related Art

In the field of absorbent articles it is well known that breathable films and certain nonwovens may be used as backsheets. In all such applications there is a compromise between one of three desired properties: water resistance, breathability, or wear resistance. When materials are combined the effects are, as expected, additive. For instance the combination of a two materials with a water resistance of 25 cm and 3 cm each will, when combined according to the prior art, produce a composite with a water resistance of approximately 28 cm.

EP-A-1,118,339 discloses an absorbent article comprising a liquid permeable topsheet, a breathable backsheet and an absorbent core, said core being intermediate said topsheet, and said backsheet, said absorbent article comprising spores which have the ability to germinate into microorganisms which exhibit antagonistic properties against undesirable strains of microorganisms.

EP-A-895,766 discloses an anatomically shaped disposable absorbent article having a front end portion and a rear end portion and a penphery, and comprising a liquid pervious topsheet, a multilayer backsheet joined to said topsheet and an absorbent core intermediate the backsheet and the topsheet. The anatomically shaped disposable absorbent article has lobes provided by the topsheet and the backsheet extending beyond the absorbent core and has a reduced tendency to form folds in the lobes during the use.

Therefore, using the teachings of the prior art, there has heretofore been no composite material which has both a high water resistance and a high breathability.

**SUMMARY OF THE INVENTION**

A composite material may be used where high breathability and high water resistance is desired, such as absorbent articles and clothing articles.

An absorbent article has a body facing side and a back side oppos the body facing side. The back side has an outer surface. The absorbent article is made up of a topsheet on the body facing side, an absorbent core between the body facing side and the back side, and a composite back sheet on the back side. The composite backsheet is made up of an apertured formed film, a meltblown layer and a wear prevention means. The apertured formed film has a male side and an opposite female side. The male side of the apertured formed film faces the absorbent core and the female side has a surface area. Female side lands are present which are more hydrophilic than other parts of the film. The meltblown layer is bonded to the female side of the formed film over more than 50% of the surface area of the female side of the formed film. The wear prevention means is on the outer surface of the back side of the absorbent article for preventing wear of the meltblown layer.

**BRIEF DESCRIPTION OF THE DRAWINGS**

Figure 1 is a cross section of an absorbent article made according to this invention.
Figure 2 is a cross section of an absorbent article made according to this invention.
Figure 3 is a cross section of a clothing article made according to this invention.

## DETAILED DESCRIPTION OF THE EXEMPLARY EMBODIMENTS OF THE INVENTION

Definitions

As used herein, the term "substantially" means that a given property or parameter may vary by about 20% from the stated value.

As used herein, the term "absorbent article" means articles that absorb and contain body exudates. More specifically, the term refers to articles which are placed against or in proximity to the body of a wearer for absorbing and containing various exudates discharged from the body. For example, "absorbent article", as used herein, includes diapers, incontinent articles, sanitary napkins, pantiliners, bandages, and other articles used to absorb body exudates.

The term "diaper" refers to a garment typically worn by infants and incontinent persons that is drawn up between the legs and fastened about the waist of the wearer. Examples of diapers from the prior art include diapers described in U.S. Pat. Re. No. 26,152, issued to Duncan, et al. on Jan. 31, 1967; U.S. Pat. No. 3,860,003 issued to Buell on Jan. 14, 1975; U.S. Pat. No. 4,610,678 issued to Weisman, et al. on Sep. 9, 1986; U.S. Pat. No. 4,673,402 issued to Weisman, et al. on Jun. 16, 1987; U.S. Pat. No. 4,695,278 issued to Lawson on Sep. 22, 1987; U.S. Pat. No. 4,704,115 issued to Buell on Nov. 3, 1987; U.S. Pat. No. 4,834,735 issued to Alemany, et al. on May 30, 1989; U.S. Pat. No. 4,888,231 issued to Angstadt on Dec. 19, 1989; and U.S. Pat. No. 4,909,803 issued to Aziz, et al. on Mar. 20, 1990.

The term "incontinent article" refers to pads, undergarments, e.g., pads held in place by a suspension system, such as a belt, or other device, inserts for absorbent articles, capacity boosters for absorbent articles, briefs, bed pads, and similar devices, whether worn by adults or other incontinent persons. Examples of incontinent articles include those disclosed in U.S. Pat. No. 4,253,461 issued to Strickland, et al. on Mar. 3, 1981; U.S. Pat. Nos. 4,597,760 and 4,597,761 issued to Buell; the above-mentioned U.S. Pat. Nos. 4,704,115; 4,909,802 issued to Ahr, et al.; U.S. Pat. No. 4,964,860 issued to Gipson, et al. on Oct. 23, 1990; and in U.S. Pat. Application Ser. Nos. 07/637,090 and 07/637,571 filed respectively by Noel, et al. and Feist, et al. on Jan. 3, 1991.

The term "sanitary napkin" refers to an article that is worn by a female adjacent to the pudendal region that is intended to absorb and contain various exudates which are discharged from the body, e.g., blood, menses, and urine. Examples of sanitary napkins are disclosed in U.S. Pat. No. 4,285,343, issued to McNair on Aug. 25, 1981; U.S. Pat. Nos. 4,589,876 and 4,687,478, issued to Van Tilburg on May 20, 1986 and Aug. 18, 1987 respectively; U.S. Pat. Nos. 4,917,697 and 5,007,906 issued to Osborn, et al. on Apr. 17, 1990 and Apr. 16, 1991, respectively; and U.S. Pat. Nos. 4,950,264 , and 5,009,653 issued to Osborn on Aug. 21, 1990 and Apr. 23, 1991, respectively; and in U.S. Pat. Application Ser. No. 07/605,583 filed Oct. 29, 1990 in the name Visscher, et al.

The term "topsheet" is used herein to refer to the layer of material in a finished absorbent article which is first contacted by liquid during an insult when the article is properly used. It is well known in the art that many finished absorbent articles employ thin sheets of nonwoven materials or perforated films as topsheet. However, this definition of topsheet is not limited to mean only sheets of nonwoven layers and perforated films but instead includes any material composition and in any shape, form, or structure which is the layer first contacted by liquid during an insult when the article is properly used.

Throughout this description, the expressions "topsheet" and "backsheet" denote the relationship of these materials or layers with respect to the absorbent core. It is understood that additional layers may be present between the absorbent core and the topsheet and backsheet, and that additional layers and other materials may be present on the side opposite the absorbent core from either the topsheet or the backsheet.

As used herein, the term "breathable" refers to a material that is permeable to water vapor having a minimum WVTR of about 300 g/m$^2$ /day. The WVTR of a fabric is water vapor transmission rate which, in one aspect, provides an indication of how comfortable a fabric would be to wear. WVTR (water vapor transmission rate) is measured as indicated below and the results are reported in grams/square meter/day. However, applications of breathable backsheets typically desirably have higher WVTRs, and breathable composites of the present invention can have WVTRs exceeding 5,000 g/m$^2$ /day, 15,000 g/m$^2$ /day, or even exceeding 20,000 g/m$^2$ /day.

As used herein, the term "SAP" means a superabsorbent polymer which, when in a substantially dry state, has the ability to spontaneously imbibe more than (20) times its own weight in aqueous fluid, for example, tap water.

As used herein, the term "non-woven web" refers to a web that has a structure of individual continuous or discrete fibers or threads which are interlaid, but not in any regular, repeating manner. Non-woven webs have been, in the past, formed by a variety of processes such as, for example, meltblowing, spunbonding, carded thermal bonding, carded hydroentangling, carded spunlacing, and other bonding techniques for fibers that are known in the art.

As used herein, the term "meltblown fibers", refers to fibers formed by extruding a molten thermoplastic material through a plurality of fine, usually circular, die capillaries as molten threads or filaments into a high velocity gas (e.g., air) stream which attenuates the filaments of molten thermoplastic material to reduce their diameter, which may be to a microfiber diameter. Thereafter, the meltblown fibers are carried by the high velocity air or plasma stream and are deposited on a collecting surface to form a web of randomly dispersed meltblown fibers.

As used herein, the term "spunbonded fibers", refers to fibers which are formed by extruding a molten thermoplastic material as filaments from a plurality of fine, usually circular, capillaries of a spinneret with the diameter of the extruded filaments then being rapidly reduced as by, for example, eductive drawing or other well-known spunbonding mechanisms.

Test Methods

The water vapor transmission rate is measured by the method set forth below. A known amount of water is put into a flanged cup. A sample is placed on the top of the cup and held securely by a retaining ring and gasket. The assembly is then weighed and recorded as the initial weight. The assembly is placed in a constant temperature (approximately 40° C) and humidity (approximately 75% Relative Humidity) chamber for 5 hours. The assembly is then removed from the chamber and allowed to equilibrate for at least 30 minutes at the temperature of the room where the balance is located. The assembly is then weighed and recorded as the final weight. The water vapor transmission rate (WVTR) is calculated and expressed in $g/m^2/day$ using the following formula:

$$WVTR = ((\text{Final weight in grams} - \text{initial weight in grams}) \times 24 \text{ hrs}) / (\text{Area of sample in meters} \times 5 \text{ hrs})$$

The water resistance value is determined by EDANA test method 120.2-02 "Recommended Test Method: Nonwovens Repellency Hydrostatic Head."

Absorbent Article Embodiments

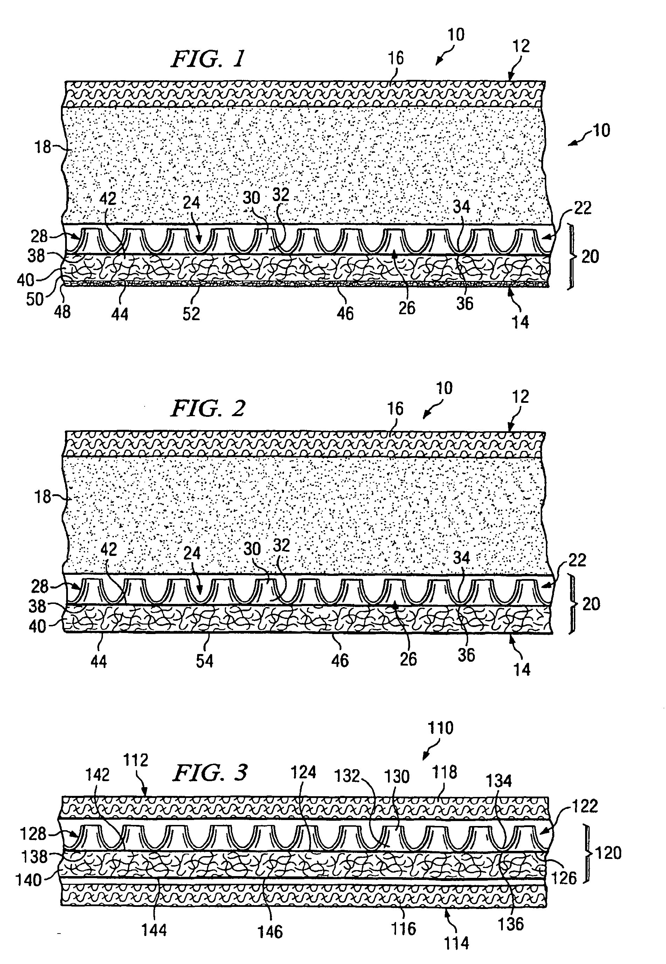

Referring to Figures 1 and 2, an absorbent article 10 is shown. The absorbent article 10 has a body facing side 12 and a back side 14. The absorbent article 10 has an absorbent core 18 with a topsheet 16 on the body facing side 12 and a composite backsheet 20 on the back side 14. Other layers may be included in this general construction.

Topsheet 16 may be of any of any design that allows fluids and exudates to pass from the body facing side 12 to the absorbent core 18. There are many known designs for topsheets 16 made of film or nonwoven materials. Multi-layer topsheets 16 are well known as well. Additionally, acquisition and distribution layers between the topsheet 16 and the absorbent core 18 may be included to improve various performance characteristics of topsheet 16 such as rewet, masking, or strikethrough.

Absorbent core 18 may be made of known materials such as natural and man made fibers, super absorbent polymers, open cell foams, or a combination of these or other absorbent materials. The design of the absorbent core 18 will depend largely on the intended use. Absorbent core 18 may include tissue layers or some other outer layer to prevent the absorbent material from escaping the core.

Composite backsheet 20 is designed to provide increased breathability and increased water resistance as compared to other backsheet materials known in the art. In this regard in a preferred embodiment the composite backsheet 20, described in more detail below, has a water resistance of between 15 mm of $H_2Om$ and 120mm of $H_2O$. In a preferred embodiment the composite backsheet 20 will have a breathability exceeding 5,000 $g/m^2/day$, 15,000 $g/m^2/day$, or even exceeding 25,000 $g/m^2/day$.

To achieve this goal composite backsheet 20 includes an apertured formed film 22 facing the absorbent core 18. Apertured formed film 22 has a male side 24 and a female side 26. Apertured formed film 22 has generally conical apertures 28 such that the male side openings 30 are smaller than female side openings 32. The cross section of conical apertures 28 may be round, hexagonal, oval, square, or any other geometric shape.

Between the conical apertures 28 on the male side 24 of the apertured formed film 22 are male side lands 34. Between the female side openings on the female side 26 of the apertured formed film. 22 are female side lands. The surface area of the female side land 36 is the surface area of the female side 26 of the apertured formed film 22. The female side lands 36 may be treated to be more hydrophilic than other portions of the film.

Apertured formed film 22 has a water resistance of 3 cm to 5 cm of water. Apertured formed film 22 a water transmission rate of 4,000 $g/m^2/day$ and 12,000 $g/m^2/day$ and a permeability of 300 cfm to 450 cfm. Apertured formed film 22 may be constructed of a variety of polymers including low density polyethylene.

Adhered to the female side 26 of the apertured formed film 22 is a meltblown layer 40. Meltblown layer 40 is attached to the female side 26 by an adhesive layer 38 which covers at least 50% of the surface area of female side 26. More preferably, adhesive layer 38 covers at least 70% of the surface area of female side 26. Most preferably, adhesive layer 38 covers at least 80% of the surface area of female side 26. This allows meltblown layer 40 to be adhered to apertured formed film 22 over at least 50% of the area of female side 26, more preferably at least 70%, and most preferably at least 80%. This high level of bonding helps to create the high level of breathability along with a high level of water resistance that has been desired in the field. In a preferred embodiment a pressure sensitive adhesive is applied at between 0.5 g/m$^2$ to 3g/m$^2$.

Adhesive layer 38 may be of any sort of pressure activated adhesive or may include thermal bonding adhesives. Adhesive layer 38 preferably includes an absorbent adhesive such as that sold by the H. B. Fuller Company in St. Paul, Minnesota. Furthermore, meltblown layer 40 may be adhered to the female side 26 by other non-point bonding means that do not change the fiber orientation of the meltblown significantly. For example, point bonding via ultrasonic bonding or hot pins would not be as effective as non-point bonding methods. Non-point bonding methods may include adhesive bonding, as discussed above, as well as thermal bonding where the fibers of meltblown layer 40 have an outer layer with a lower meting point to assist in thermal bonding. Other methods of non-point bonding are known as well.

The meltblown layer 40 has a body facing side 42, which is adhered to the female side 26 of the apertured formed film 22, and a back side 44 opposite the body facing side 42. A preferred embodiment has a meltblown layer 40 with a basis weight of 7 g/m$^2$ to 50 g/m$^2$. Meltblown layer 40 may be comprised of a variety of materials, including polypropylene fibers, polyethylene fibers, bi-component fibers, and hydrophobic fibers. A preferred embodiment has a meltblown layer with a water vapor transmission rate between 10,000 g/m$^2$/day and 12,000 g/m$^2$/day. A preferred embodiment has a meltblown layer with a water resistance of 10 cm to 60 cm of water. In a preferred embodiment the meltblown layer 40 exhibit a median wet pore radius of no more than $15\mu m$ (microns).

The composite backsheet 20 will exhibit a water resistance at least 25% greater than the sum of the water resistance of the apertured formed film 22 and the water resistance of the meltblown layer 40.

A wear prevention means 46 is applied to the back side 44 of the meltblown layer 40. As shown in Figure 1, wear prevention means 46 may include a nonwoven layer 48 having a body facing side 50 and a back side 52. Body facing side 50 of nonwoven layer 48 is secured to the back side 44 of meltblown layer 40. Nonwoven layer 48 is designed to be highly breathable, but wear resistant to protect meltblown layer 40 from wear, and particularly pilling.

Nonwoven layer 48 may be secured to meltblown layer 40 with pressure activated adhesives, thermally activated adhesives or other attachment means that are known in the art. Again, a water absorbent adhesive is preferable in this application since it will allow the air to pass and will trap any fluid that may be condensating on the external layer. A preferred embodiment has a nonwoven layer 48 with a basis weight of 10g/m$^2$ to 25 g/m$^2$**.**

As shown in Figure 2, wear prevention means 46 may include a chemical treatment to the back side 44 of meltblown layer 40. In particular, a wear resistant coating 54 may be applied to the back side 44 of meltblown layer 40 to protect meltblown layer from wear, and particularly pilling. Examples of such wear resistant coatings 54 may include a latex adhesive or other wear resistant coatings, such as ultraviolet curable silicone and Teflon aerosol spray, that would not negatively effect the breathability of composite backsheet 20 substantially. In a preferred embodiment wear resistant coating 54 is applied at between 0.05 g/m$^2$ and 4 g/m$^2$.

Wear prevention means 46 may also include the selection of a particular type of fiber to form the meltblown layer 40. For example, a latex fiber may be used as the primary fiber in the meltblown layer 40 thereby preventing wear and pilling of the meltblown layer

Clothing Embodiments

As shown in Figure 3, the composite backsheet 20, used in the absorbent articles 10 shown in Figures 1 and 2, may also be incorporated into a clothing article 110 to provide a highly breathable composite layer 120 that also has high water resistance. While the structures are similar, the positioning is changed somewhat. In the absorbent article 10 fluid was to be retained in the absorbent core and air was allowed to pass through the composite backsheet 20. In the clothing article 110 water is to be maintained outside while air is allowed to pass through the composite layer 120.

Clothing article 110 has an outward facing side 112 and a body facing side 114. Clothing article 110 may have an outer layer 118 and an inner layer 116. The outer layer 118 and inner layer 116 will be breathable and chosen for their drape, texture, and appearance.

Between optional outer layer 118 and optional inner layer 116 is composite layer 120. Composite layer 120 is designed to provide increased breathability and increased water resistance at a low cost as compared to other clothing materials known in the art. To achieve this goal composite layer 120 includes an apertured formed film 122 facing an optional outer layer 1 Apertured formed film 122 has a male side 124 and a female side 126. Apertured formed film 122 has generally conical apertures 128 such that the male side openings 130 are smaller than female side openings 132. The cross section of conical apertures 128 may be round, hexagonal, oval, square, or any other geometric shape.

Between the conical apertures 128 on the male side 124 of the apertured formed film 122 are male side lands 134. Between the female side openings on the female side 126 of the apertured formed film 122 are female side lands. The surface area of the female side lands 136 is the surface area of the female side 126 of the apertured formed film 122. The female side lands 136 may be treated to be more hydrophilic than other portions of the film.

Adhered to the female side 126 of the apertured formed film 122 is a meltblown layer 140. Meltblown layer 140 is attached to the female side 126 by an adhesive layer 138 which covers at least 50% of the surface area of female side 126. More preferably, adhesive layer 138 covers at least 70% of the surface area of female side 126. Most preferably, adhesive layer 138 covers at least 80% of the surface area of female side 126. This allows meltblown layer 140 to be adhered to apertured formed film 122 over at least 50% of the area of female side 126, more preferably at least 70%, and most preferably at least 80%. This high level of bonding helps to create the high level of breathability along with a high level of water resistance that has been desired in the field.

Adhesive layer 138 may be of any sort of pressure activated adhesive or may include thermal bonding adhesives. Adhesive layer 138 preferably includes a water absorbent adhesive such as that sold by the H. B. Fuller Company in St. Paul, Minnesota. Furthermore, meltblown layer 140 may be adhered to the female side 126 by other non-point bonding means. For example, point bonding via ultrasonic bonding or hot pins would not be as effective as non-poinf bonding methods. Non-point bonding methods may include adhesive bonding, as discussed above, as well as thermal bonding where the fibers of meltblown layer 140 have an outer layer with a lower meting point to assist in thermal bonding. Other methods of non-point bonding are known as well.

The meltblown layer 140 has a outward facing side 142, which is adhered to the female side 126 of the apertured formed film 122, and a body facing side 144 opposite the outward facing side 142.

A wear prevention means 146 may be applied to the body facing side 144 of the meltblown layer 140. Wear prevention means 146 may include a nonwoven or chemical treatment as discussed above. Wear prevention means 146 may also include the selection of a particular type of fiber to form the meltblown layer 140. For example, a latex fiber may be used as the primary fiber in the meltblown layer 140 thereby preventing wear and pilling of the meltblown layer.

Manufacturing Method

The composite backsheet 20 of Figure 1 and Figure 2 is formed by providing a vacuum formed film 22 with a male side 24 and a female side 26. A meltblown layer 40 is bonded to the female side 26 of the vacuum formed film 22 over at least 50% of the area of vacuum formed film 22, preferably greater than 70%, and more preferably greater than 80%. Preferably the bonding will not significantly alter the fiber alignment of the meltblown layer 40. In that regard, an adhesive layer 38 may be used to adhesively bond the meltblown layer 40 to the vacuum formed film 22.

A wear prevention means 46 is added to the back side 44 of meltblown layer 40. The wear prevention means 46 may include the addition of a nonwoven layer 48, as shown in Figure 1, the addition of a wear resistant coating 54, as shown in Figure 2, or the use of more wear resistant fiber in the meltblown layer 40.

The same method may be used to manufacture composite 120.

Conclusion

While the present invention has been described in detail with respect to specific embodiments thereof, it will be appreciated that those skilled in the art may readily conceive alterations to, variations of, and equivalents to those embodiments. The scope of the present invention should therefore be determined by the appended claims and equivalents thereto.

**Claims**

1. An absorbent article having a body facing side and a back side opposite the body facing side, the back side having an outer surface, the absorbent article comprising:

   a topsheet on the body facing side;
   an absorbent core between the body facing side and the back side; and
   a composite back sheet on the back side having

   an apertured formed film with a male side and an opposite female side, the male side facing the absorbent core, the female side having a surface area;
   female side lands which are more hydrophilic than other parts of the film;
   a meltblown layer bonded to the female side of the formed film over more than 50% of the surface area of the female side of the formed film; and
   a wear prevention means on the outer surface of the back side of the absorbent article for preventing wear of the meltblown layer.

2. The absorbent artide of claim 1 wherein the apertured formed film includes generally conical tapered apertures with a larger opening on the female side of the formed film and a smaller opening on the male side of the formed film.

3. The absorbent article of claim 1 wherein the meltblown layer is bonded to the formed film over an area greater than 70% of the surface area of the female side of the formed film.

4. The absorbent article of claim 1 wherein the meltblown layer is bonded to the formed film over an area greater than 80% of the surface area of the female side of the formed film.

5. The absorbent article of claim 1 wherein the formed film has a water resistance of 3 cm to 5 cm of water.

6. The absorbent article of claim 1 wherein the formed film has a water vapor transmission rate of 4,000 $g/m^2/day$ and 12,000 $g/m^2/day$.

7. The absorbent article of claim 1 wherein the formed film is a low density polyethylene.

8. The absorbent article of claim 1 wherein the permeability of the formed film is 300 cfm to 450 cfm.

9. The absorbent article of claim 1 wherein the meltblown layer is adhesively bonded to the female side of the formed film.

10. The absorbent article of claim 1 wherein the meltblown layer is adhesively bonded with a pressure sensitive adhesive applied at between 0.5 $g/m^2$ and 3 $g/m^2$.

11. The absorbent article of claim 1 wherein the meltblown layer is adhesively bonded with an adhesive containing between 10% and 90% absorbent adhesive, by weight.

12. The absorbent article of claim 1 wherein an absorbent adhesive is either applied between the meltblown layer and the formed film or applied on the male side of the formed film.

13. The absorbent article of claim 1 wherein the meltblown layer is comprised of polypropylene fibers.

14. The absorbent article of claim 1 wherein the meltblown layer has a basis weight of 7 $g/m^2$ to 50 $g/m^2$.

15. The absorbent article of claim 1 wherein the meltblown layer has a water vapor transmission rate between 10,000 $g/m^2/day$ and 12,000 $g/m^2/day$.

16. The absorbent article of claim 1 wherein the meltblown layer has a water resistance of 10 cm to 60 cm of water.

17. The absorbent article of claim 1 wherein the meltblown layer is comprised of hydrophobic fibers.

18. The absorbent article of claim 1 wherein the wear resistant means is a nonwoven layer bonded to the meltblown layer opposite the formed film such that the nonwoven layer forms the outer surface of the back side of the absorbent article.

19. The absorbent articles of claim 18 wherein the nonwoven layer is a carded web that is thermally or hydro-entangled bonded.

20. The absorbent article of claim 18 wherein the nonwoven layer is spunbonded.

21. The absorbent article of claim 18 wherein the nonwoven layer has a basis weight of 13 $g/m^2$ to 15 $g/m^2$.

22. The absorbent article of claim 1 wherein the wear resistant means is a wear resistant coating applied to the meltblown layer opposite the formed film such that the coated meltblown layer forms the outer surface of the back side of the absorbent article.

23. The absorbent article of claim 22 wherein the wear resistant coating is latex adhesive.

24. The absorbent article of claim 22 wherein the wear resistant coating is a curable silicone.

**25.** The absorbent article of claims 22 wherein the wear resistant coating is applied at between 0.05 g/m$^2$ and 5 g/m$^2$.

**26.** A method for manufacturing a highly breathable, water resistant, and wear resistant backsheet comprising the steps of:

> providing an apertured formed film with a male side and a female side, the female side having a surface area; providing female side lands which are more hydrophilic than other parts of the film
> bonding a first side of a meltblown layer to at least 50% of the surface area on the female side of the formed film; and
> applying a wear prevention means to a second side of the meltblown opposite the formed film.

**27.** The method of claim 26 wherein the applying of a wear prevention means is the thermal bonding of a nonwoven layer.

**28.** The method of claim 26 wherein the applying of a wear prevention means is the application of a wear resistant coating.

**29.** The method of claim 26 wherein the meltblown layer is bonded to at least 70% of the surface area on the female side of the formed film.

**30.** The method of claim 26 wherein the meltblown layer is bonded to at least 80% of the surface area on the female side of the formed film.

**31.** A highly breathable, water repellent, wear resistant composite for resisting moisture flow from a first direction and allowing air flow in a second direction opposite the first direction, the composite comprising:

> an apertured formed film with a male side and an opposite female side, the male side facing the first direction and the female side having a surface area; female side lands which are more hydrophilic than other parts of the film;
> a meltblown layer bonded to the female side of the formed film over more than 50% of the surface area of the female side of the formed film; and
> a wear prevention means applied to the meltblown layer opposite the formed film such that the coated meltblown layer faces the second direction.

**32.** The composite of claim 31 wherein the wear prevention means is a wear resistant coating.

**33.** The composite of claim 31 wherein the wear prevention means is a nonwoven layer bonded to the meltblown layer.


**Patentansprüche**

**1.** Saugfähiger Gegenstand mit einer dem Körper zugewandten Seite sowie einer Rückseite, die gegenüber der dem Körper zugewandten Seite liegt, wobei die Rückseite eine äußere Oberfläche aufweist, wobei der saugfähige Gegenstand umfasst:

> eine obere Lage auf der dem Körper zugewandten Seite;
> einen saugfähigen Kern zwischen der dem Körper zugewandten Seite und der Rückseite; und
> eine Lage auf der Rückseite aus Verbundstoff:

>> einen mit Öffnungen gebildeten Film mit einer männlichen Seite und einer gegenüberliegenden weiblichen Seite, wobei die männliche Seite dem saugfähigen Kern zugewandt ist, und die weibliche Seite einen Oberflächenbereich aufweist;
>> Übergangsflächen (lands) der weiblichen Seite, welche hydrophiler sind als andere Abschnitte des Films;
>> eine Meltblown-Schicht, die mit der weiblichen Seite des gebildeten Films über mehr als 50% des Oberflächenbereichs der weiblichen Seite des gebildeten Films hinweg verbunden ist; und
>> ein Verschleiß-verhinderndes Mittel auf der äußeren Oberfläche der Rückseite des saugfähigen Gegenstands zum Verhindern von Verschleiß der Meltblown-Schicht.

**2.** Saugfähiger Gegenstand nach Anspruch 1, wobei der mit Öffnungen gebildete Film im Allgemeinen konisch zulaufende Öffnungen mit einer größeren Öffnung auf der weiblichen Seite des gebildeten Films und einer kleineren Öffnung auf der männlichen Seite des gebildeten Films aufweist.

3.  Saugfähiger Gegenstand nach Anspruch 1, wobei die Meltblown-Schicht mit dem gebildeten Film über einen Bereich verbunden ist, der größer als 70% des Oberflächenbereichs der weiblichen Seite des gebildeten Films ist.

4.  Saugfähiger Gegenstand nach Anspruch 1, wobei die Meltblown-Schicht mit dem gebildeten Film über einen Bereich verbunden ist, der größer als 80 % des Oberflächenbereichs der weiblichen Seite des gebildeten Films ist.

5.  Saugfähiger Gegenstand nach Anspruch 1, wobei der gebildete Film eine Beständigkeit gegen Wasser von 3 cm bis 5 cm Wasser aufweist.

6.  Saugfähiger Gegenstand nach Anspruch 1, wobei der gebildete Film eine Wasserdampf-Übertragungsrate von 4000 g/m$^2$/Tag und 12000 g/m$^2$/Tag aufweist.

7.  Saugfähiger Gegenstand nach Anspruch 1, wobei der gebildete Film ein Polyethylen mit niedriger Dichte ist.

8.  Saugfähiger Gegenstand nach Anspruch 1, wobei die Permeabilität des gebildeten Films 300 cfm (cubic feet per minute) (Kubikfuß pro Minute) bis 450 cfm beträgt.

9.  Saugfähiger Gegenstand nach Anspruch 1, wobei die Meltblown-Schicht haftend mit der weiblichen Seite des gebildeten Films verbunden ist.

10. Saugfähiger Gegenstand nach Anspruch 1, wobei die Meltblown-Schicht haftend mit einem druckempfindlichen Haftmittel, das zwischen 0,5 g/m$^2$ und 3 g/m$^2$ aufgebracht ist, verbunden ist.

11. Saugfähiger Gegenstand nach Anspruch 1, wobei die Meltblown-Schicht haftend mit einem Haftmittel verbunden ist, das zwischen 10 Gew.-% und 90 Gew.-% saugfähiges Haftmittel aufweist.

12. Saugfähiger Gegenstand nach Anspruch 1, wobei ein saugfähiges Haftmittel entweder zwischen der Meltblown-Schicht und der gebildeten Schicht angebracht ist oder auf der männlichen Seite des gebildeten Films angebracht ist.

13. Saugfähiger Gegenstand nach Anspruch 1, wobei die Meltblown-Schicht aus Polypropylen-Fasern zusammenge-setzt ist.

14. Saugfähiger Gegenstand nach Anspruch 1, wobei die Meltblown-Schicht eine flächenbezogene Masse von 7 g/m$^2$ bis 50 g/m$^2$ aufweist.

15. Saugfähiger Gegenstand nach Anspruch 1, wobei die Meltblown-Schicht eine Wasserdampf-Übertragungsrate zwi-schen 10000 g/m$^2$/Tag und 12000 g/m$^2$/Tag aufweist.

16. Saugfähiger Gegenstand nach Anspruch 1, wobei die Meltblown-Schicht eine Beständigkeit gegen Wasser von 10 cm bis 60 cm Wasser aufweist.

17. Saugfähiger Gegenstand nach Anspruch 1, wobei die Meltblown-Schicht aus hydrophoben Fasern besteht.

18. Saugfähiger Gegenstand nach Anspruch 1, wobei das Mittel, das gegen Verschleiß beständig ist, eine nicht-gewebte Schicht ist, die mit der Meltblown-Schicht gegenüber dem gebildeten Film so verbunden ist, dass die nicht-gewebte Schicht die äußere Oberfläche der Rückseite des saugfähigen Gegenstands bildet.

19. Saugfähiger Gegenstand nach Anspruch 18, wobei die nicht-gewebte Schicht ein Krempelvlies ist, der thermisch oder hydroverwickelt (hydro-entangled) verbunden ist.

20. Saugfähiger Gegenstand nach Anspruch 18, wobei die nicht-gewebte Schicht vliesverfestigt ist.

21. Saugfähiger Gegenstand nach Anspruch 18, wobei die nicht-gewebte Schicht eine flächenbezogene Masse von 13 g/m$^2$ bis 15 g/m$^2$ aufweist.

22. Saugfähiger Gegenstand nach Anspruch 1, wobei das Mittel, das gegen Verschleiß beständig ist, eine Beschichtung ist, die gegen Verschleiß beständig ist, wobei die Beschichtung so an der Meltblown-Schicht gegenüber dem gebildeten Film angebracht ist, dass die beschichtete Meltblown-Schicht die äußere Oberfläche der Rückseite des saugfähigen Gegenstands bildet.

23. Saugfähiger Gegenstand nach Anspruch 22, wobei die gegen Verschleiß beständige Beschichtung ein Latexhaftmittel ist.

24. Saugfähiger Gegenstand nach Anspruch 22, wobei die gegen Verschleiß beständige Beschichtung ein vulkanisierbares Silikon ist.

25. Saugfähiger Gegenstand nach Anspruch 22, wobei die gegen Verschleiß beständige Beschichtung zwischen 0,05 g/m$^2$ und 5 g/m$^2$ aufgebracht ist.

26. Verfahren zur Herstellung einer äußerst atmungsfähigen, gegen Wasser beständigen sowie gegen Verschleiß beständigen Lage auf der Rückseite umfassend die Schritte:

Bereitstellen eines mit Öffnungen gebildeten Films mit einer männlichen Seite und einer weiblichen Seite, wobei die weibliche Seite einen Oberflächenbereich aufweist;
Bereitstellen von Übergangsflächen (lands) der weiblichen Seite, welche hydrophiler sind als andere Abschnitte des Films;
Verbinden einer ersten Seite einer Meltblown-Schicht mit mindestens 50 % des Oberflächenbereichs auf der weiblichen Seite des gebildeten Films; und
Anbringen eines Mittels, das gegen Verschleiß beständig ist, an einer zweiten Seite der Meltblown-Schicht gegenüber des gebildeten Films.

27. Verfahren nach Anspruch 26, wobei das Anbringen eines Verschleiß-verhindernden Mittels die thermische Bindung einer nicht-gewebten Schicht darstellt.

28. Verfahren nach Anspruch 26, wobei das Anbringen eines Verschleiß-verhindernden Mittels das Anbringen einer Beschichtung darstellt, die gegen Verschleiß beständig ist.

29. Verfahren nach Anspruch 26, wobei die Meltblown-Schicht mit mindestens 70 % des Oberflächenbereichs auf der weiblichen Seite des gebildeten Films verbunden wird.

30. Verfahren nach Anspruch 26, wobei die Meltblown-Schicht mit mindestens 80 % des Oberflächenbereichs auf der weiblichen Seite des gebildeten Films verbunden wird.

31. Äußerst atmungsfähiger, wasserabweisender, gegen Verschleiß beständiger Verbundstoff, der gegen Feuchtigkeitsstrom aus einer ersten Richtung beständig ist und Luftstrom in eine zweite Richtung entgegen der ersten Richtung ermöglicht, wobei der Verbundstoff umfasst:

einen mit Öffnungen gebildeten Film mit einer männlichen Seite und einer gegenüberliegenden weiblichen Seite, wobei die männliche Seite der ersten Richtung zugewandt ist und die weibliche Seite einen Oberflächenbereich aufweist;
Übergangsflächen (lands) der weiblichen Seite, welche hydrophiler sind als andere Abschnitte des Films;
eine Meltblown-Schicht, die mit der weiblichen Seite des gebildeten Films mit mehr als 50% des Oberflächenbereichs der weiblichen Seite des gebildeten Films verbunden ist; und
ein Verschleiß-verhinderndes Mittel, das an der Meltblown-Schicht gegenüber des gebildeten Films so angebracht ist, dass die beschichtete Meltblown-Schicht der zweiten Richtung zugewandt ist.

32. Verbundstoff nach Anspruch 31, wobei das Verschleiß-verhindernde Mittel eine Beschichtung ist, die gegen Verschleiß beständig ist.

33. Verbundstoff nach Anspruch 31, wobei das Verschleiß-verhindernde Mittel eine nicht gewebte Schicht ist, die mit der Meltblown-Schicht verbunden ist.

**Revendications**

1. Article absorbant comportant une face tournée vers le corps et une face formant dossier, opposée à la face tournée vers le corps, la face formant dossier ayant une surface externe, l'article absorbant comprenant :

   une feuille supérieure sur la face tournée vers le corps ;
   un noyau absorbant entre la face tournée vers le corps et la face formant dossier ; et
   une feuille formant dossier, composite, sur la face formant dossier ayant :

       un film façonné perforé ayant une face mâle et une face femelle, opposée, la face mâle étant tournée vers le noyau absorbant, la face femelle ayant une surface spécifique ;
       des plats latéraux femelles qui sont davantage hydrophobes que d'autres parties du film ;
       une couche obtenue par extrusion-soufflage liée à la face femelle du film façonné sur plus de 50 % de la surface spécifique de la face femelle du film façonné ; et
       un moyen de protection contre l'usure sur la surface externe de la face formant dossier de l'article absorbant pour empêcher l'usure de la couche obtenue par extrusion-soufflage.

2. Article absorbant selon la revendication 1, dans lequel le film façonné perforé comprend des ouvertures effilées généralement coniques ayant une plus grande ouverture sur la face femelle du film façonné et une plus petite ouverture sur la face mâle du film façonné.

3. Article absorbant selon la revendication 1, dans lequel la couche obtenue par extrusion-soufflage est liée au film façonné sur une superficie supérieure à 70 % de la surface spécifique de la face femelle du film façonné.

4. Article absorbant selon la revendication 1, dans lequel la couche obtenue par extrusion-soufflage est liée au film façonné sur une superficie supérieure à 80 % de la surface spécifique de la face femelle du film façonné.

5. Article absorbant selon la revendication 1, dans lequel le film façonné a une résistance à l'eau de 3 cm à 5 cm d'eau.

6. Article absorbant selon la revendication 1, dans lequel le film façonné a un débit de transmission de la vapeur d'eau de 4 000 g/m$^2$/jour et de 12 000 g/m$^2$/jour.

7. Article absorbant selon la revendication 1, dans lequel le film façonné est un polyéthylène basse densité.

8. Article absorbant selon la revendication 1, dans lequel la perméabilité du film façonné est de 300 cfm (pieds cubes par minute) à 450 cfm.

9. Article absorbant selon la revendication 1, dans lequel la couche obtenue par extrusion-soufflage est liée de façon adhésive à la face femelle du film façonné.

10. Article absorbant selon la revendication 1, dans lequel la couche obtenue par extrusion-soufflage est liée de façon adhésive au moyen d'un adhésif sensible à la pression appliqué en une quantité comprise entre 0,5 g/m$^2$ et 3 g/m$^2$.

11. Article absorbant selon la revendication 1, dans lequel la couche obtenue par extrusion-soufflage est liée de façon adhésive au moyen d'un adhésif contenant entre 10 % et 90 % d'adhésif absorbant, en poids.

12. Article absorbant selon la revendication 1, dans lequel un adhésif absorbant est soit appliqué entre la couche obtenue par extrusion-soufflage et le film façonné, soit appliqué sur la face mâle du film façonné.

13. Article absorbant selon la revendication 1, dans lequel la couche obtenue par extrusion-soufflage est composée de fibres de polypropylène.

14. Article absorbant selon la revendication 1, dans lequel la couche obtenue par extrusion-soufflage a une masse surfacique comprise entre 7 g/m$^2$ et 50 g/m$^2$.

15. Article absorbant selon la revendication 1, dans lequel la couche obtenue par extrusion-soufflage a un débit de transmission de la vapeur d'eau compris entre 10 000 g/m$^2$/jour et 12 000 g/m$^2$/jour.

**16.** Article absorbant selon la revendication 1, dans lequel la couche obtenue par extrusion-soufflage a une résistance à l'eau de 10 cm à 60 cm d'eau.

**17.** Article absorbant selon la revendication 1, dans lequel la couche obtenue par extrusion-soufflage est composée de fibres hydrophobes.

**18.** Article absorbant selon la revendication 1, dans lequel le moyen résistant à l'usure est une couche non tissée liée à la couche obtenue par extrusion-soufflage à l'opposé du film façonné de manière que la couche non tissée forme la surface externe de la face formant dossier de l'article absorbant.

**19.** Article absorbant selon la revendication 18, dans lequel la couche non tissée est un voile cardé qui est lié thermiquement ou par hydroliage.

**20.** Article absorbant selon la revendication 18, dans lequel la couche non tissée est obtenue par filage-nappage.

**21.** Article absorbant selon la revendication 18, dans lequel la couche non tissée a une masse surfacique comprise entre 13 g/m$^2$ et 15 g/m$^2$.

**22.** Article absorbant selon la revendication 1, dans lequel le moyen résistant à l'usure est un revêtement résistant à l'usure appliqué sur la couche obtenue par extrusion-soufflage à l'opposé du film façonné de manière que la couche obtenue par extrusion-soufflage, revêtue, forme la surface externe de la face formant dossier de l'article absorbant.

**23.** Article absorbant selon la revendication 22, dans lequel le revêtement résistant à l'usure est un adhésif au latex.

**24.** Article absorbant selon la revendication 22, dans lequel le revêtement résistant à l'usure est une silicone durcissable.

**25.** Article absorbant selon la revendication 22, dans lequel le revêtement résistant à l'usure est appliqué en une quantité comprise entre 0,05 g/m$^2$ et 5 g/m$^2$.

**26.** Procédé pour fabriquer une feuille formant dossier hautement respirante, résistante à l'eau et résistante à l'usure, comprenant les étapes consistant à :

fournir un film façonné perforé ayant une face mâle et une face femelle, la face femelle ayant une surface spécifique ;
fournir des plats latéraux femelles qui sont davantage hydrophobes que d'autres parties du film ;
lier une première face d'une couche obtenue par extrusion-soufflage à au moins 50 % de la surface spécifique sur la face femelle du film façonné ; et
appliquer un moyen de protection contre l'usure sur une deuxième face de la couche obtenue par extrusion-soufflage, opposée au film façonné.

**27.** Procédé selon la revendication 26, dans lequel l'application d'un moyen de protection contre l'usure est la liaison thermique d'une couche non tissée.

**28.** Procédé selon la revendication 26, dans lequel l'application d'un moyen de protection contre l'usure est l'application d'un revêtement résistant à l'usure.

**29.** Procédé selon la revendication 26, dans lequel la couche obtenue par extrusion-soufflage est liée à au moins 70 % de la surface spécifique sur la face femelle du film façonné.

**30.** Procédé selon la revendication 26, dans lequel la couche obtenue par extrusion-soufflage est liée à au moins 80 % de la surface spécifique sur la face femelle du film façonné.

**31.** Composite résistant à l'usure, hautement respirant, hydrofuge, pour résister à l'écoulement d'humidité depuis une première direction et permettre l'écoulement d'air dans une deuxième direction, opposée à la première direction, le composite comprenant :

un film façonné perforé ayant une face mâle et une face femelle, opposée, la face mâle étant tournée vers la première direction et la face femelle ayant une surface spécifique ;

des plats latéraux femelles qui sont davantage hydrophobes que d'autres parties du film ;
une couche obtenue par extrusion-soufflage liée à la face femelle du film façonné sur plus de 50 % de la surface spécifique de la face femelle du film façonné ; et
un moyen de protection contre l'usure appliqué sur la couche obtenue par extrusion-soufflage à l'opposé du film façonné de manière que la couche obtenue par extrusion-soufflage, revêtue, soit tournée vers la deuxième direction.

32. Composite selon la revendication 31, dans lequel le moyen de protection contre l'usure est un revêtement résistant à l'usure.

33. Composite selon la revendication 31, dans lequel le moyen de protection contre l'usure est une couche non tissée liée à la couche obtenue par extrusion-soufflage.

FIG. 1

FIG. 2

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 60334451 B
- EP 1118339 A
- EP 895766 A
- US 26152 A, Duncan
- US 3860003 A, Buell
- US 4610678 A, Weisman
- US 4673402 A, Weisman
- US 4695278 A, Lawson
- US 4704115 A, Buell
- US 4834735 A, Alemany
- US 4888231 A, Angstadt
- US 4909803 A, Aziz
- US 4253461 A, Strickland
- US 4597760 A

- US 4597761 A, Buell
- US 4909802 A, Ahr
- US 4964860 A, Gipson
- US 637090 A
- US 07637571 A
- US 4285343 A, McNair
- US 4589876 A
- US 4687478 A, Van Tilburg
- US 4917697 A
- US 5007906 A, Osborn
- US 4950264 A
- US 5009653 A, Osborn
- US 60558390 A, Visscher